Europäisches Patentamt

European Patent Office

Office européen des brevets

(11) Publication number: **0 263 634 B1**

# EUROPEAN PATENT SPECIFICATION

(45) Date of publication of patent specification: **11.08.93**  (51) Int. Cl.5: **C12M 1/00**, C12M 3/00

(21) Application number: **87308623.5**

(22) Date of filing: **29.09.87**

(54) **Culture medium supplying method and culture system.**

(30) Priority: **29.09.86 JP 228380/86**
          **19.08.87 JP 205510/87**

(43) Date of publication of application:
**13.04.88 Bulletin 88/15**

(45) Publication of the grant of the patent:
**11.08.93 Bulletin 93/32**

(84) Designated Contracting States:
**CH DE FR GB LI NL SE**

(56) References cited:
**EP-A- 0 112 812**
**WO-A-87/06610**
**CA-A- 1 170 596**
**US-A- 2 923 669**

**BIOTECHNOLOGY & BIOENGINEERING, vol. 26, no. 5, May 1984, pages 503-507, John Wiley & Sons, Inc., New York, US; J.M.STRAND et al.: "Amodified matrix perfusion-microcarrier bead cell culture system. I. Adaptation of the matrix perfusion system for growth of human foreskin fibroblasts"**

(73) Proprietor: **SUZUKI SHOKAN CO. LTD.**
**No. 1 Koujimachi 3-chome Chiyoda-ku Tokyo 102(JP)**

(72) Inventor: **Mori, Junichi**
**Suzuki Shokan Co. Ltd. No. 1 Koujimachi 3-chome**
**Chiyoda-ku Tokyo 102(JP)**
Inventor: **Abe, Masaaki**
**Suzuki Shokan Co. Ltd. No. 1 Koujimachi 3-chome**
**Chiyoda-ku Tokyo 102(JP)**

(74) Representative: **Crampton, Keith John Allen et al**
**D. YOUNG & CO., 21 New Fetter Lane London EC4A 1DA (GB)**

## Description

This invention relates to the supply of a culture medium into a culture vessel for cultivating cells and to systems for carrying out such supply.

Recent thorough investigations of systems for reproducing biochemical cells in artificial vessels have resulted in systems that can produce an artificial environment for the biochemical reaction in a living body and make it possible to produce useful substances such as immunoglobulin continuously in large quantities and more efficiently than hitherto. Such substances had previously been produced by cultivating inoculation cultures in the living bodies of animals such as mice. Among known artificial systems are jar fermentors, roller bottles and membrane cultivating equipment.

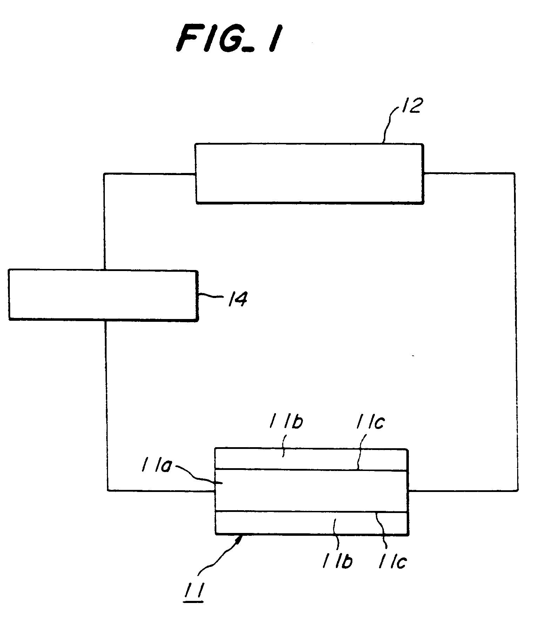

In the accompanying drawings, Fig. 1 is a block diagram schematically illustrating one example of a known arrangement of a culture system using membranes, and including a culture vessel (for example, a hollow-fibre filter) using the membranes and a culture medium tank from which a predetermined culture medium is supplied into the culture vessel where the desired cells are cultivated.

In Fig. 1, a hollow-fibre culture vessel is in the form of filter 11, which mainly consists of a flow passage 11a for culture medium from culture medium tank 12, and culture chambers 11b in which the cells are cultivated. Moreover, the flow passages 11a and the culture chambers 11b are partitioned by membranes capable of excluding predetermined molecular weights. Such partitions 11c serve to prevent cells cultivated in the culture chambers 11b from entering the flow passage 11a but permit the culture medium flowing in the flow passage 11a to be supplied into the culture chambers 11b and waste matter to be removed from the culture chambers 11b into the flow passage 11a.

Moreover, the culture medium stored in the culture medium tank 12 has been adjusted to have predetermined values of such parameters as pH and dissolved oxygen concentration (DO). The culture medium tanks of some known systems include means for automatically adjusting such parameters.

A driving source 14 serves to supply the culture medium from the culture medium tank 12 into the culture vessel 11. Therefore, the culture medium enters one side of the flow passage 11a of the culture vessel 11 and leaves on the other side of the culture vessel 11. As the driving source, peristaltic pumps, bellow pumps or magnetic pumps have been used.

When using a peristaltic pump, sliding means scrapes the outer surfaces of a silicon tube of the pump to drive culture medium in the silicon tube to supply culture medium to vessel 11; when using a bellows pump, culture medium stored in a bellows is fed out of the bellows by its extension and contraction to supply culture medium to vessel 11; when using a magnetic pump, a rotor in the pump is rotated by magnetic force from outside the pump to supply culture medium to vessel 11.

However, when a peristaltic pump is used, the tube is often damaged after use for a long period of time, with the result either that the culture medium is not supplied to the culture vessel but flows elsewhere, or that the culture chambers are contaminated. In order to avoid such problems, it is necessary to displace the sliding means to new positions as a regular maintenance operation. Moreover, because the sliding means scrapes the tube, worn tube material is mixed into the culture medium, which must therefore be filtered.

Furthermore, the constructional characteristics of a peristaltic pump cause pulsations in the medium being fed into the culture vessel, thereby disturbing the supply of the culture medium into the culture vessel.

In using a bellows pump or magnetic pump, particular maintenance and operation of the pump are needed although the disadvantages of the peristaltic pump are somewhat mitigated.

In previously used culture systems, particularly those using ultrafiltration membranes, the direction of flow of the culture medium in the flow passage in the culture vessel is fixed. Therefore, when anchored cells are used for cultivation, the cells anchored to the culture chamber upstream of the flow of the medium into the chamber are subjected to culture medium having the desired adjusted parameters such as pH and DO, but the cells anchored downstream of the flow tend to include waste matter produced by metabolism of the upstream cells; and when suspended cells are used, they are likely to be carried downstream by the flow. A locally uneven cultivating environment is thus produced so that the cultivating density of the cultivated substances becomes uneven.

The present invention provides a method of supplying a culture medium into a culture vessel in which the culture vessel is connected between two culture medium supply and collection reservoirs that both contain culture medium and a gas phase, the pressure of the gas phase in one such reservoir being different from that in the other whereby culture medium flows from one reservoir to the other, and whereby suitably changing gas pressures in the reservoirs can reverse the direction of flow of the culture medium. The method of the invention is capable of substantially avoiding the disadvantages described above and of

EP 0 263 634 B1

supplying the culture medium stably at constant flow for a long period of time.

Feeding culture medium under gas pressure means subjecting culture medium in a reservoir to a higher pressure to force it out of the reservoir into a system at a lower pressure than in the reservoir, and includes the case where the system is made lower in pressure than the reservoir.

In carrying out the method of the invention, it is preferable to periodically change the direction of flow of the culture medium fed through the culture vessel, the period for changing the direction of flow being determined by the nature of the substance being cultivated, for example, tissue cells forming skin and internal organs of animals.

In accordance with a second embodiment of the invention, a culture system comprises at least one culture vessel, a culture medium supply section and a gas pressure supply section;

the culture medium supply section having at least two, that is, a first and a second, culture-medium-supply-and-collection reservoirs, in which each of these reservoirs is for containing a gas phase and a culture medium phase and is provided with at least one gas port and at least one culture medium port;

the culture vessel having two culture medium ports, the culture medium port of the first culture-medium-supply-and-collection reservoir being connected to one of the culture medium ports of the culture vessel through a culture medium flow passage, and the other culture medium port of the culture vessel being connected to the culture medium port of the second culture-medium-supply-and-collection reservoir through a culture medium flow passage; and

the gas pressure supply section comprising a gas-pressure-producing section for generating gas at at least two different pressures and having at least two gas ports for feeding the two different-pressure gases, one of the gas ports of the gas-pressure-producing section being connected to the gas port of the first reservoir through a gas flow passage, and the other gas port of the gas-pressure-producing section being connected to the gas port of the second reservoir through a gas flow passage.

The means for supplying culture medium comprises a culture-medium-adjusting reservoir having means for adjusting parameters such as pH and DO, first and second reservoirs for supplying, collecting and replenishing the culture medium, flow passages for connecting these reservoirs, and switching-over means for selecting the flow passages according to requirements. Flow passages will be provided to connect the culture-medium-adjusting reservoir to each end of the culture vessel, with appropriate flow passage switching-over means for making effective either of the flow passages, and to connect the culture vessel and the first and second culture-medium supply and collection reservoirs with flow-passage switching-over means to switch over to collect the culture medium from the culture vessel in one culture-medium-supply-and-collection reservoir when the flow passage between the other such reservoir and the culture medium adjusting tank is effective. The switching-over means is preferably controlled by a control section described below.

Furthermore, the gas-pressure-supply means comprises a gas-pressure producer having first and second pressure chambers, and a compressor for producing pressures in the first and second pressure chambers different from each other such that for example the pressure in the first pressure chamber is higher than in the second pressure chamber. Moreover, the gas-pressure-supply means comprises gas supply flow passages for feeding the gas from the respective pressure chambers into required locations of the culture system, including the means for applying culture medium, and switching-over means for switching over these gas flow passages.

By keeping the pressure difference at a constant value, the flow rate of the culture medium can be maintained at a constant value corresponding to the pressure difference, thereby preventing pulsations in the culture medium. Further, by changing the pressure difference, the amount of the culture medium supplied can be controlled.

Moreover, by changing the nature of the gas, the amount of gas dissolved in the culture medium can be easily controlled.

When one of the periodical changes in the direction of flow of the culture medium takes place, the change stirs the medium in the culture chambers of the culture vessel and makes more uniform the cultivating conditions in the culture chambers.

In more detail, when a passage is formed through the first pressure chamber, the first culture medium supply and collection reservoir, the culture medium adjusting reservoir, the culture vessel, the second culture medium supply and collection reservoir and the second pressure chamber, the culture medium is fed under pressure from the first to the second pressure chamber by way for the first culture medium supply and collection reservoir, then through the culture-medium adjusting reservoir and the culture vessel into the second culture medium supply and collection reservoir.

In order that the invention may be more clearly understood, preferred embodiments will be described by way of example, with reference to the accompanying drawings, in which:-

3

Fig. 1 (as already mentioned) is a block diagram schematically illustrating an examplary arrangement of a known culture system;

Figs. 2A and 2B are block diagrams illustrating respective parts of the principal portion of one embodiment of a culture system according to the invention;

Fig. 3 is a block diagram illustrating a modification of a gas pressure supply system according to the invention;

Figs. 4A and 4B are block diagrams illustrating respective parts of the principal portion of another embodiment of a culture system according to the invention.

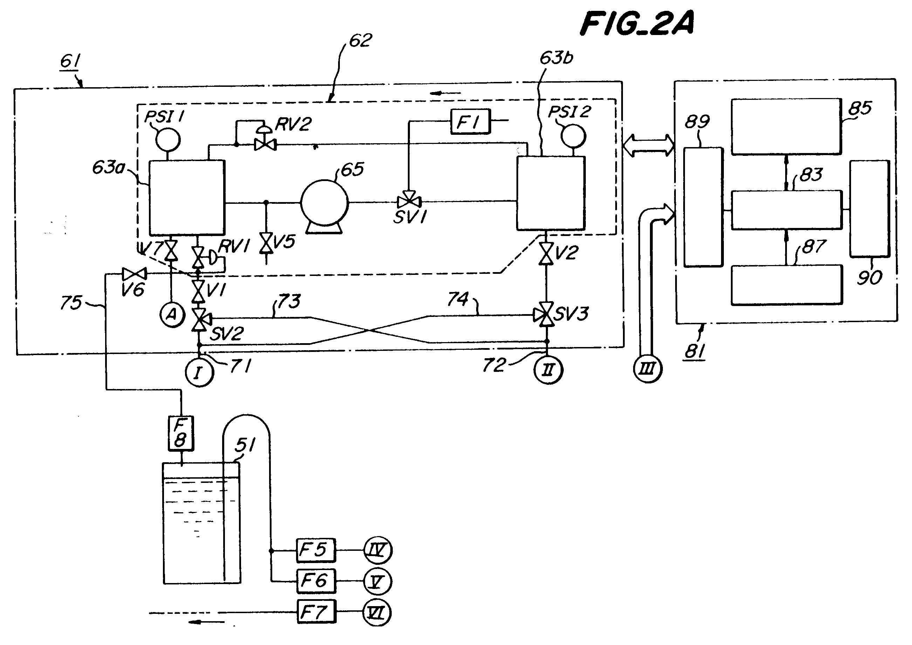

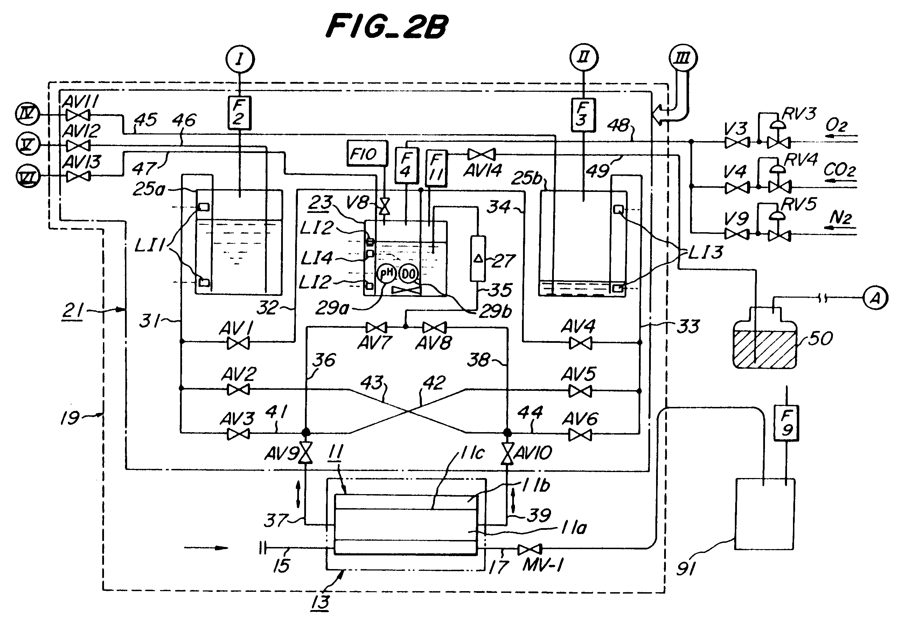

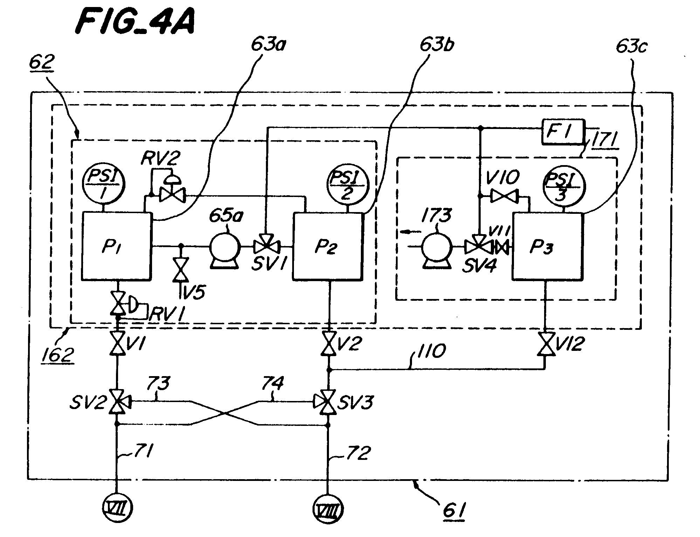

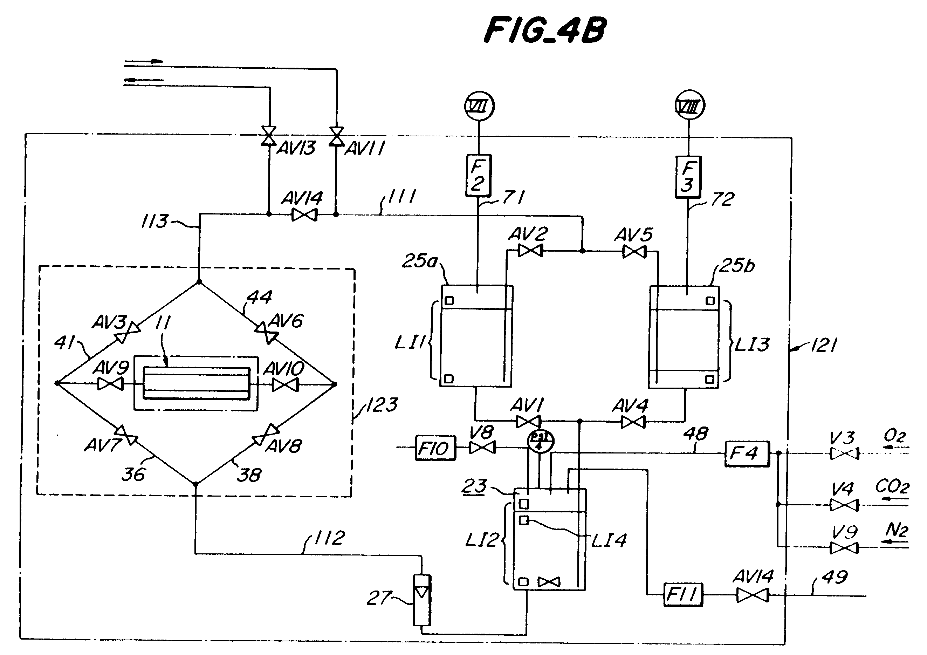

The block diagrams are drawn schematically to the extent necessary for the invention to be understood, omitting sections for operations such as sterilization, cleaning and maintenance for the sake of clarity. The arrangement of the respective components is not limited to those shown in the drawings, and the encircled Roman numbers I to VI in Fig. 2A and VII and VIII In Fig. 4A are connected to the corresponding encircled Roman numerals in Figs. 2B and 4B respectively.

In Figs. 2A and 2B, a culture vessel 11 (Fig. 2B) is in the form of a hollow-fibre filter using a known hollow fibre. As explained with reference to Fig. 1, the filter 11 has a passage 11a for a culture medium, culture chambers 11b and partition walls 11c between them for excluding material of a predetermined molecular weight. In this culture system a first temperature control means 13, for example, a water jacket, maintains the filter at the desired environmental temperature. The filter 11 further comprises an inlet 15 for supplying an inoculation culture and an outlet 17 for removing products from the filter 11. A product receiving tank 91 is connected through a manually operable value MV1 to the outlet 17.

A culture medium supply section 21 comprises a culture medium adjusting reservoir 23 for adjusting such parameters as pH and DO, supply and collection reservoir 25a and 25b, culture medium flow passages provided between the culture vessel 11, the culture medium adjusting reservoir 23 and the reservoir 25a and 25b in predetermined relationship (later described in detail). In order to detect the upper and lower limit levels of the stored culture medium, the adjusting reservoir 23 includes level gauges LI2, and the reservoirs 25a and 25b include level gauges LI1 and LI3, respectively.

The switching-over means are of valves AV1, AV2 and AV3 (later described in detail) provided in a predetermined manner in the flow passages in the culture medium supply section 21, and a control section 81 including means for closing and opening these valves. The valves are driven by gas pressure from a gas pressure supply section 61 according to instructions from the control section 81, but the driving of the valves is not limited to this feature. For example, they could be magnetically driven.

The reservoir 25a and the reservoir 23 are connected through flow passage 31, valve AV1 and flow passage 32. The reservoir 25b and the reservoir 23 are connected through flow passage 33, valve AV4 and flow passage 34.

The reservoir 23 and one end of the flow passage 11a of the culture vessel 11 are connected by flow passage 35, which has a flow meter 27, flow passage 36, which has a valve AV7, and flow passage 37, which has a valve AV9. Moreover, the reservoir 23 and the other end of the flow passage 11a are connected by flow passage 35, which has a flow meter 27, flow passage 38, which has a valve AV8, and flow passage 39, which has a valve AV10.

The flow passages 31 and 36 are connected by valve AV3 and flow passage 41; the flow passages 33 and 36 are connected by valve AV5 and flow passage 42; the flow passages 31 and 38 are connected by valve AV2 and flow passage 43; and the flow passages 33 and 38 are connected by valve AV6 and flow passage 44.

A culture medium replenishing reservoir 51 is connected through a sterilization filter F5, a valve AV11 and a flow passage 45 to the reservoir 25b and is further connected through a sterilization filter F6, a valve AV12 and a flow passage 46 to the reservoir 25a.

The culture-medium-adjusting reservoir 23 is supplied through a flow passage 48 with oxygen, carbon dioxide or nitrogen and is further supplied through a flow passage 49 with an alkaline solution (e.g. 7.5% by weight aqueous sodium bicarbonate) through a reservoir 50 as shown in Fig. 2B, thereby adjusting pH or DO in the reservoir 23 by the gas and chemical liquid. The reservoir 23 includes a sensor 29a for a pH meter and a sensor 29b for a dissolved oxygen meter as shown in Fig. 2B. The introduction of the gas will change the pressure in the reservoir 23, with the result that the surface level of the medium in it is also changed. In order to provide for such an extraordinary change in surface level, the reservoir 23 comprises a level gauge LI4 and a valve V8 for venting. The culture-medium-adjusting reservoir 23 also serves as a buffer for restraining change in flow rate when switching over the flow passages.

Disused culture medium can be removed from the culture medium adjusting tank 23 through a flow passage 47, a valve AV13 and a sterilization filter F7.

In this embodiment, moreover, the culture medium supply section and the culture vessel 11 as above described are accommodated in a constant temperature bath 19 having second temperature control means.

The gas pressure supply section 61 includes a gas-pressure-producing section 62 having first and second pressure chambers 63a and 63b and a compressor 65 for generating different gas pressures in the chambers 63a and 63b. An external gas, for example, the air is introduced through a filter F1 and a magnetic three-way valve SV1 into the compressor 65, where the gas is compressed and fed into and stored in the first pressure chamber 63a. A remaining passage of the three-way valves SV1 is connected to the second pressure chamber 63b. The pressure in the first pressure chamber is therefore higher than that of the second pressure chamber.

Moreover, the first and second pressure chambers 63a and 63b are provided with pressure gauges PSI1 and PSI2, both of which have pressure switches, and are connectable by a primary pressure regulating valve RV2. These pressure chambers can be set at predetermined pressures in order to supply gas pressures from these pressure chambers for feeding the culture medium under pressure difference. When the pressure in the first pressure chamber 63a reaches a predetermined value, the pressure in the chamber 63a is regulated by primary pressure-regulating valve RV2. If the pressure in the first pressure chamber 63a becomes lower than the predetermined value, the magnetic three-way valve SV1 is switched over to the air-introducing side (filter F1 side) to replenish the air in the pressure chamber 63a until the desired pressure is reached. When the pressure in the second pressure chamber 63b becomes lower than the predetermined value, the three-way valve SV1 is also switched over to the air-introducing side to introduce external air into the chamber 63a. Thereafter, an excess of air in the chamber 63a is fed through the primary pressure regulating valve RV2 into the second pressure chamber 63b.

Between the first pressure chamber 63a and the first tank 25a of the culture medium supply section 21, there is provided a gas glow passage 71 having a pressure-regulating valve RV1, a magnetic valve V1, a magnetic three-way valve SV2 and a sterilization filter F2. Between the second pressure chamber 63b and the second tank 25b, there is provided a gas flow passage 72 having a valve V2, a three-way valve SV3 and a filter F3. Moreover, the three-way valve SV2 and the gas flow passage 72 are connected by a gas flow passage 73, and the three-way valve SV3 and the gas flow passage 71 are connected by a gas flow passage 74.

With this arrangement, one of the reservoirs 25a and 25b is connected to the first pressure chamber 63a, while the other is connected to the second pressure chamber 63b, so that a pressure difference occurs between the reservoirs 25a and 25b to enable the culture medium to be fed under pressure difference into either of the tanks.

By switching over the three-way valves SV2 and SV3, the connection of reservoirs 25a and 25b to pressure chambers 63a and 63b can be changed over.

The gas for feeding the culture medium under pressure has been said to be air in the above embodiment. However, it is often the case that the amount of dissolved gas in the culture medium greatly affects the proliferation of cells or formation of products, depending upon the substances involved.

Too much or too little oxygen can be dissolved in the culture medium, i.e. more or less than the equilibrium amount under the atmospheric pressure, and there are culture materials for which the former case is the better environment, and those for which the latter is better. When such substances are cultivated, it is preferable to use the appropriate amount of dissolved oxygen in the culture medium.

In order to fulfil any such requirement, the gas used for feeding the medium under pressure difference is chosen so that for example the equilibrium amount of oxygen dissolved in the medium can be easily controlled by changing the gas or components of it. With a culture material preferring dissolved oxygen in excess of the equilibrium amount under atmospheric pressure, air mixed with oxygen at a high concentration is supplied through filter F1 into the gas pressure producing section shown in Figs. 2A and 2B. With a culture substance preferring dissolved oxygen in amount is less than the equilibrium amount, air having a low concentration of oxygen, e.g. obtained by dilution with nitrogen, can be supplied.

The section for controlling the respective components can be constructed according to known control techniques. That shown at 81 in Fig. 2a comprises a microprocessor 83, a memory device 85 for storing data such as feeding directions of culture media and programmes of culture conditions, an input unit 87 for instructing mode selection and change of the feeding directions and culture conditions, input and output (I/O) ports for reading pressure data of the first and second pressure chambers, flow rates of culture media, data of pH, DO and other parameters, data of amounts of culture media in the respective reservoirs, and outputting instruction signals for modifying parameters such as pressure, pH and DO and operating the respective values on the basis of these data, and a display unit 90 for displaying various messages.

The operation of the culture system can be effected in the following sequence (1) - (6), although the present invention is of course not limited to this sequence. Moreover, the following numerical parameters are only by way of example, and could be modified as required.

(1) Sterilization

(2) Supply of culture medium to the culture medium supply section

(3) Adjusting the culture medium

(4) Cells inoculation into the culture vessel

(5) Supplying the culture medium to the culture vessel

(6) Sampling during period of cultivation

In this embodiment, moreover, hollow-fibre filter cartridges having a molecular weight cut off of 30,000 and manufactured by Grace Co. under the trade name of "Vitafiber II" are used for the culture vessel.

(1) Sterilization

Before the cultivation of cells, the reservoirs 23, 25a and 25b and the flow passages inside the zone bounded by sterilization filters F2, F3, F5, F6, F7, F9, F10 and F11 associated with the culture medium supply section 21 are sterilized by steam at any suitable temperature, for example, 120°C for a predetermined time, for example, 30 minutes.

(2) Supply of culture medium to the culture medium supply section.

Then, the culture medium is supplied from the culture-medium-replenishing reservoir into the culture medium supply section. In this case, the culture medium is first replenished from the replenishing reservoir 51 into one of the supply and collection reservoirs, for example, 25a. Thereafter, the culture medium is fed through the culture-medium-adjusting reservoir 23 into the other of reservoirs 25a and 25b.

Therefore, the valve V1 is opened and the gas, pressurized or adjusted by pressure-adjusting valve RV1, is supplied from the first pressure chamber 63a through the flow passage 75 and the sterilization filter F8 into the reservoir 51. On the other hand, the valve V2, the three-way valve SV3 and valves AV6, AV8 and AV1 are operated respectively to make effective the passage of the second pressure chamber 63b, the gas flow passage 72, the reservoir 25b, the flow passage 33, valve AV6, the flow passages 44 and 38, the valve AV8, the flow passage 35, the flow meter 27, the reservoir 23, the flow passage 32, the valve AV1 and the flow passage 31, thereby forming a pressurized feeding system through the culture-medium-replenishing reservoir 51, the reservoirs 25a and 25b, and the reservoir 23. After the formation of the feeding system, the valve AV12 is opened to supply the culture medium from the supply reservoir 51 through the flow passage 46 into the first reservoir 25a, and the culture medium is fed under pressure difference from the first reservoir 25a through the adjusting reservoir 23 into the second reservoir 25b. The amount of the culture medium in the second reservoir 25b is monitored by means of the level gauge LI3. When it is detected that the liquid surface of the supplied culture medium has reached the lower limit level of the level gauge LI3, the valves AV1, AV6 and AV8 are closed. In order to feed the culture medium under pressure difference from the supply tank 51 into the first tank 25a continuously, the three-way valve SV3 is switched over to the side of the flow passage 74 to make effective the passage of the second pressure chamber 63b, the valve V2, the three-way valve SV3, the flow passages 74 and 71, the filter F2, the first tank 25a, the flow passage 46 and the valve AV12. The liquid surface of the culture medium in the first tank 25a is monitored by the level gauge L1. When it is detected that the liquid surface has arrived at the upper limit level of the gauge LI1, the valves AV12 and AV6 are closed.

Thereafter, the valve V1 and the three-way valve SV2 are actuated to supply the gas pressure in the first pressure chamber 63a, adjusted to a predetermined pressure by the pressure adjusting valve RV1, into the reservoir 25a through the gas flow passage 71. (The gas pressure adjusted to the predetermined pressure of the pressure adjusting valve RV1 is referred to sometimes as "first gas pressure".) Moreover, the valve V2 and the three-way valve SV3 are actuated to supply the gas pressure in the second pressure chamber 63b to the reservoir tank 25b through the gas flow passage 72. (The gas pressure in the second pressure chamber to be supplied into the second tank 25b is referred to sometimes as "second gas pressure".) Further, the valves AV1, AV8 and AV6 are opened to feed the culture medium in the reservoir 25a under pressure difference into the reservoir 25b through the culture-medium-adjusting reservoir 23. The amount of the culture medium in the reservoir 23 is monitored by means of the level gauge LI2.

(3) Adjusting the culture medium

In the culture-medium-adjusting reservoir 23, respective parameters such as the pH and DO of the medium are measured by sensors 29a and 29b. These measured results are fed into control section 81 and

6

if the values of these parameters are outside the range of values suitable for culture cells, the culture medium is treated so as to bring the values to the desired levels. For example, oxygen can be replenished through the flow passage 48 into the reservoir 23, or if less oxygen is required, nitrogen is added through the passage 48. If a higher pH is needed, an alkaline solution in the reservoir tank 50 is supplied through the flow passage 49 into the reservoir 23; if a lower pH is needed, $CO_2$ gas is supplied through the flow passages 48 into the reservoir 23 or an acid aqueous solution may be supplied through passage 48. Moreover, as the culture-medium-adjusting reservoir 23 is arranged in the constant temperature bath 19, the culture medium in the adjusting tank 23 is controlled substantially at a predetermined temperature. During the cultivation operation, the temperature of the medium in the culture vessel is controlled with higher accuracy by means of the first temperature control means 13.

After the pH and other parameters of the medium have been adjusted in the reservoir 23, the medium is not immediately supplied to the culture vessel 11 (by keeping the valves AV9 and AV10 closed) and is circulated in the culture medium supply section 21 to stabilize it.

To stabilize the culture medium, the medium in the reservoir 23 is first fed under pressure difference into the reservoir 25b through the flow passage 35, the valves AV8 and AV6 and the flow passage 33. The upper level of the culture medium in the reservoir 25b is monitored by the level gauge LI3. The valve AV6 is then closed and the valve AV4 is opened. At this moment, the three-way valves SV2 and SV3 are opened to switch over the gas flow passages so that the second gas pressure is supplied from the second pressure chamber 63b into the reservoir 25a and the first gas pressure is supplied from the first pressure chamber 63a into the reservoir 25b. Moreover, the valve AV1 is closed, and the valves AV7 and AV3 are opened. Therefore, the culture medium is then fed under pressure difference from the reservoir 25b into the reservoir 23 in which the parameters such as the pH are adjusted in the manner as above described. Then, the adjusted culture medium is fed under pressure difference into the reservoir through the flow passage 35, the valve AV7, the flow passage 36, the valve AV3 and the flow passage 31.

The culture medium is cyclically circulated between the medium adjusting reservoir 23 and the reservoirs 25a and 25b in the manner as above described to bring the temperature, pH and DO of the culture medium to the desired predetermined values.

(4) Cells inoculation into the culture vessel

Then, for example, innoculation cells are poured into the culture chamber 11b through the inlet 15 of the hollow fibre 11 under an environment that has been treated so as not to contaminate the inoculation cells.

(5) Supplying the culture medium to the culture vessel

The supply of the culture medium to the culture vessel will be explained with reference to the case above, in the supply section, the first gas pressure is supplied into the reservoir 25a and the second gas pressure is supplied into the reservoir 25b, while the culture medium returning from the vessel 11 is stored or collected in the reservoir 25b (as shown in Fig. 2).

In this case, to cause the culture medium to flow in two directions in the culture vessel, the culture medium in the first reservoir 25a is fed, for a first period under pressure difference, through the reservoir 23 by way of the flow passage 35, the valve AV7, the flow passage 36, the valve AV9, the flow passage 37, the culture vessel 11, the flow passage 39, the valves AV10 and AV6, the flow passage 33 and the reservoir 25b for a period of time during cell culture. For a second period, the culture medium in the first reservoir 25a is fed under pressure difference through the reservoir 23 by way of the flow passage 35, the valve AV8, the flow passage 38, the valve AV10, the flow passage 39, the culture vessel 11, the flow passage 37, the valve AV9, the flow passage 42, the valve AV5, the flow passage 33 and the reservoir 25b. In this manner, the culture medium is caused to flow successively in opposite directions, with the number and frequency of switching over operations being appropriately determined according to the culture cells. The direction of the culture medium fed into the culture vessel is changed periodically.

Moreover, when the culture medium supplied to the culture vessel is collected in the reservoir 25b and its upper surface reaches the upper limit level therein, the culture medium flow passages as above described are switched over so that the second gas is supplied to the reservoir 25a, while the first gas is supplied to the reservoir 25b to collect the culture medium returning from the culture vessel 11 in the reservoir 25a. In this manner, the culture medium supply can be effected in the manner described above.

Furthermore, the culture medium can be fed under pressure difference in one direction without switching over the flow directions of the culture medium in the culture vessel.

(6) Sampling of culture and like substances during the period of cultivation

Sampling is carried out to estimate the yield of product with a view to optimizing it.

In a mode of supplying the culture medium to the culture vessel, the valve AV9 (or AV10) downstream of the flow of the culture medium in the culture vessel 11 is closed, and the valve MV1 between the outlet 17 of the culture vessel 11 and a product-receiving reservoir 91 is opened. As a result, a pressure difference occurs between the culture-medium-adjusting reservoir 23 and the product-receiving reservoir 91, so that the produce in the culture chamber 11b is fed together with the culture medium under pressure difference into the product-receiving reservoir 91. The cels sampled in this manner are observed with microscopes, other products with suitable reagents.

Moreover, on the basis of such an observation of the culture medium, change in pH and consumed amount of DO, the time to change the culture medium is determined. Further, part of the culture medium is sampled by passage through the reservoir 23, the flow passage 47, the valve AV13 and the sterilization filter F7. Metabolites such as glucose and lactic acid in the sampled culture medium are estimated to find the amounts of components consumed and changes in waste material, in addition to the above observations. The results of the estimation may be used for determining the exchange time of the culture medium.

Various modifications of the above embodiments are contemplated.

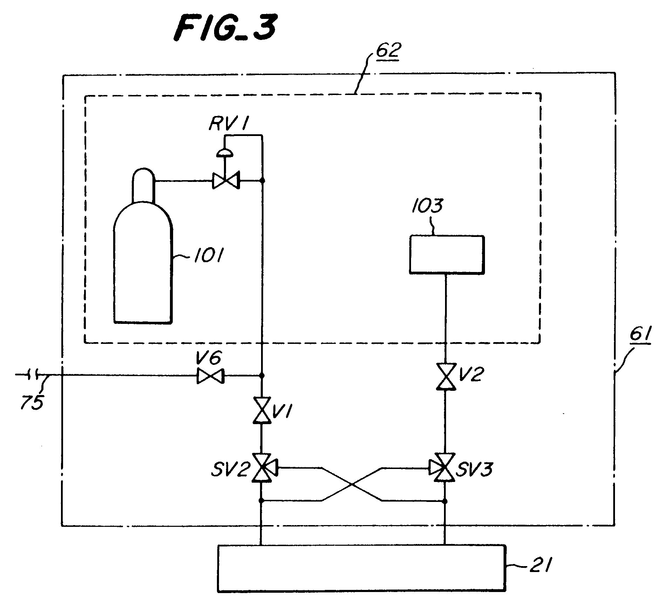

For example, the gas-pressure-producing section 62 may be constructed in a simple manner, as shown in Fig. 3, where it includes a high-pressure gas bomb 101, with a pressure regulator and a valve V2 on the lower pressure side being directly connected to the atmospheric pressure without providing a low pressure chamber. With this arrangement, it is preferable to provide a filter having open mesh at an inlet for introducing the air to protect the sterilization filters F2 and F3. In a factory having an installation supplied with a high-pressure gas, for example, air or nitrogen, the gas pressure may be obtained from such an installation.

Moreover, the gas to be introduced into the gas-supply section is not limited to the air. Other suitable gases, for xample, nitrogen, argon or mixtures of these gases may be used.

In the above embodiment, the culture medium supply section has been explained provided with the culture-medium-adjusting reservoir 23. However, the latter is not essential and pH and DO may instead be adjusted in the culture medium supply and collection tank or any appropriate flow passages. Further, the culture system of the above embodiment has been shown provided with means for adjusting the pH, DO and temperature of the culture medium, but it may also be provided with means for adjusting other parameters, for example, dissolved concentration of $CO_2$, depending upon the material to be cultivated.

Moreover, the supply of the culture medium and the removal of products may be effected by suction with negative pressure. In an embodiment for supplying the culture medium from the reservoir 51 into the reservoir 25a by suction, the flow passage 75 between the sterilization filter F8 and the valve V6 is removed and once end of the filter F8 on the side oppsotie to the replenishing tank 51 is opened or exposed to the atmosphere.

The three-way valve SV1 is switched over to the side of the second pressure chamber 63b and the compressor 65 is operated. Then the valve V5 is opened and the valve V1 closed, and the air in the second pressure chamber 63a is forced out of the system through the valve V5 to bring the second pressure chamber 63b into negative pressure. Therefore, the respective valves are switched over so that the system of the second pressure chamber 63b, the valve V2, the three-way valve SV3, the flow passages 74 and 71, the filter F2, the first tank 25a and the flow passage 46 becomes effective. The valve AV12 is then opened so that the culture medium in the replenishing tank 51 is fed under pressure difference into the first reservoir 25a by suction.

After starting the feeding under pressure difference, the liquid level of the culture medium in the reservoir 25a is monitored by the level gauge LI1 of the tank. When the gauge LI1 detects that the medium has reached its upper limit, the valves AV12 and V2 are closed.

Then, the gas-pressure-producing section is brought into a condition similar to the culture-medium-supplying condition above described. Thereafter, the respective valves are switched over so that the effective flow system consists of the first pressure chamber 63a, the valves RV1, V1 and SV2, the flow passage 71, the filter F2, the reservoir 25a, the flow passage 31, the valve AV1, the flow passage 32, the culture-medium-adjusting reservoir 23, the flow meter 27, the flow passage 35, the valve AV8, the flow passages 38 and 44, the valve AV6, the flow passage 33, the reservoir 25b, the filter F3, the flow passage 72, the valves SV3 and V2 and the second pressure chamber 63b. Therefore, the culture medium in the first tank 25a is supplied through the adjusting tank 23 into the second tank 25b. The amount of the culture medium fed into the second tank 25b is monitored by the level gauge LI3. When the liquid surface has reached the lower limit, the above flow system is completely closed so that the gas-producing section is returned to the suction mode as above described, whereby the culture medium in the culture-medium-replenishing reservoir 51 is replenished into the reservoir 25a in the manner described above. The amount of the culture medium in the first tank 25a is monitored by the level gauge LI1. When the liquid surface of the medium has arrived at the upper limit, the valve AV12 is closed. The liquid surfaces of the medium replenished in the reservoirs 25a, 25b and 23 as above described are shown in Fig. 2B. The replenishment of the culture medium can be effected by suction in this manner.

The culture medium supply section and the gas-pressure-producing section are not limited to the above embodiments and various modifications may be made depending upon supplying methods of the culture medium.

For example, the culture system may be constituted as shown in Fig. 4 to provide for the features that a culture medium is continuously introduced into the culture system from outside, continuously supplied to the culture vessel and continuously drained out of the culture system, that a culture medium is continuously supplied into the culture vessel, while part of the culture medium is circulated in the culture system or drained out of the culture system, and that a culture medium is circulated in the culture system while part of the culture medium is drained out of the culture system or is replenished from outside of the culture system.

Figs. 4A and 4B are schematic block diagrams of respective parts of principal portions of a culture system capable of supplying and circulating the culture medium in this manner. In Figs. 4A and 4B, like components are designated by the same reference numerals as those in Figs. 2A and 2B, while components not essential for an understanding of the embodiment, for example, control section 81 and other detailed parts are omitted.

In Fig. 4A, a gas-pressure-producing section 162 comprises a negative-gauge pressure-producing section 171 in addition to the component designated by 62 in Fig. 2A. The negative-gauge-pressure-producing section 171 comprises a vacuum pump 173, a third pressure chamber 63c, a three-way valve SV4, magnetic valves V10 and V11, and a pressure indicator PSI 3 with a pressure switch. The vacuum pump 173 is controlled depending upon a set value of the pressure indicator PSI 3 to maintain the gas pressure in the third pressure chamber 63c at a desired negative gauge pressure. The third pressure chamber 63c is connected through a gas flow passage 110 having a magnetic valve V12 to the flow passage 72 (already explained) between the valves V2 and the three-way valve SV3.

The compressor, the vacuum pump and the valves of the gas-pressure-producing section 162 are operated so that pressures $P_1$, $P_2$ and $P_3$ in the first, second and third pressure chambers 63a, 63b and 63c are maintained in the relation $P_1 > P_2 >$ atmospheric pressure $> P_3$.

With the culture system including the gas-pressure-producing section 162, the three-way valves SV2 and SV3 are switched over to provide four conditions: that the gas pressure $P_1$ is supplied to the reservoir 25a and the gas pressure $P_2$ to the reservoir 25b, that $P_2$ is supplied to the reservoir 25a and $P_1$ to the reservoir 25b, that $P_1$ is supplied to the reservoir 25a and $P_3$ to the reservoir 25b, and that $P_3$ is supplied to the reservoir 25a and $P_1$ to the reservoir 25b.

A modified culture medium supply section 121 is shown in Fig. 4B. The culture medium supply section 121 is fundamentally similar to that shown in Fig. 2B with exception that arrangements of the culture medium flow passages and valves are different from those shown in Fig. 2B.

In this embodiment, the culture medium is supplied through a flow passage 111 shown in Fig. 4B into the reservoir 25a or 25b. The culture medium in this reservoir is further caused to flow through a passage of the culture-medium-adjusting tank 23, the flow meter 27, a flow passage 112, a flow direction switch-over bypath block 123 (referred to sometimes The hereinafter "switch-over block"), and a flow passage 113.

The switch-over block 123 is constructed as follows:

A loop-shaped flow passage is formed by a flow passage 36 having a valve AV7, a flow passage 38 having a valve AV8, a flow passage 41 having a valve AV3, and a flow passage 44 having a valve AV6. The flow passage 112 is connected to a join between the flow passages 36 and 38, and the flow passage 113 is connected to a join between the flow passages 41 and 44. Moreover, between the join of the flow passages 36 and 41 and the join of the flow passages 38 and 44, there is provided a system consisting of a valve AV9, the culture vessel 11 and a valve AV10. Therefore, these valves are switched over so that in this switch-over block, the flow directions of the culture medium can be switched over and the culture medium circulated without supplying new culture medium into the culture vessel 11 in a similar manner to that explained with reference to Fig. 2B. These passages are for supplying the culture medium into the culture vessel 11 in the following order (a) the flow passage 112, the valves AV7 and AV9, the culture vessel 11, the valves AV10 and AV6 and the flow passage 113, or (b) the flow passage 112, the valves AV8 and AV10, the culture vessel 11, the valves AV9 and AV3, and the flow passage 113. Moreover, the other passages are bypass passages for bypassing the culture vessel 11 through the flow passage 112, the valves AV7 and AV3, the flow passage 113 or flow passage 112, the valves AV8 and AV6, and the flow passage 113.

In this embodiment, the switch-over block is constructed as follows. The first and second flow passages 36 and 41 having the valves AV7 and AV3 are connected in series with each other. The third and fourth passages 38 and 44 having the valves AV8 and AV6 are connected in series with each other. The first and second flow passages 36 and 41, which are connected in series, and the third and fourth flow passages 38 and 44, which are connected in series, are connected in parallel in the flow passage consisting of the flow passages 112 and 113. The fifth flow passage having the valve AV9 is connected between one end of the culture vessel 11 and the join between the first and second flow passages 36 and 41. The sixth flow passage having the valve AV10 is connected between the other end of the culture vessel and the connection between the third and second flow passages 38 and 44.

Examples of the culture system shown in Figs. 4A and 4B will be explained with regard to the mode of feeding culture media hereinafter.

Example 1

In this case a culture medium is continuously introduced into the culture system from the outside, continuously fed into the culture vessel 11, and continuously drained from the outlet of the culture system.

The respective valves are operated so that the gas pressure $P_1$ is supplied to the reservoir 25a and the gas pressure $P_3$ is supplied to the reservoir 25b. Then the passage comprising the valve AV11, the flow passage 111, the valve AV5, and the reservoir 25b is made effective, so that culture medium is fed under suction from the outside of the culture system through a culture medium supply replenishing line on the primary side of the valve AV11 into the reservoir 25b. On the other hand, the passage comprising reservoir 25a, the valve AV1, the culture-medium-adjusting tank 23, the flow meter 27, the flow passage 112, the switch-over block 123, the flow passage 113 and the valve AV3 is made effective, so that the culture medium in the reservoir 25a is drained under positive pressure through the above passage out of the culture system.

Moreover, the respective valves are opened so that the gas pressure $P_3$ is supplied to the reservoir 25a and the gas pressure $P_1$ is supplied to the reservoir 25b. Then the passage comprising the valve AV11, the flow passage 111, the valve AV2 and the reservoir 25a is made effective, so that the culture medium is fed under suction from the outside of the culture system into the reservoir 25a. On the other hand, the passage comprising the reservoir 25b, the valve AV4, the culture-medium-adjusting tank 23, the flow meter 27, the flow passage 112, the switch-over block 123, the flow passage 113 and the valve AV13 is made effective, so that the culture medium in the reservoir 25b is drained under positive pressure through the above passage including the switch-over block out of the culture system.

The two passages for supplying and draining the culture medium as above described are used by switching them over under the monitoring of the level gauges LI1 - LI3. The supply and drain of the culture medium according to Example 1 is carried out in this manner.

Example 2

In this case a culture medium introduced into the culture system is continuously circulated.

The introduction of the culture medium into the culture system is effected in the same manner as explained in Example 1.

The respective valves are operated so that the gas pressure $P_1$ is supplied to the reservoir 25a and the gas pressure $P_2$ is supplied to the reservoir 25b. Moreover, the passage formed by the reservoir 25a, the valve AV1, the culture- medium- adjusting chamber 23, the flow meter 27, the flow passage 112, the switch-over block 123, the flow passage 113, the valve AV14, the flow passage 111, the valve AV5 and the reservoir 25b is made effective, so that the culture medium in the first reservoir 25a is fed under positive pressure into the reservoir 25b through the above passage including the switch-over block 123.

The respective valves are operated so that the gas pressure $P_2$ is supplied to the reservoir 25a and the gas pressure $P_1$ is supplied to the reservoir 25b. Moreover, the passage formed by the reservoir 25b, the valve AV4, the culture-medium-adjusting chamber 23, the flow meter 27, the flow passage 112, the switch-over block 123, the flow passage 113, the valve AV14, the flow passage 111, the valve AV2, and the first tank 25a is made effective, so that the culture medium in the reservoir 25b is fed under positive pressure into the reservoir 25a through the above passage including the switch-over block 123.

The two culture medium supply and drain passages above described are switched over under monitoring by the level gauges LI1 - LI3 to circulate the culture medium and feed it under pressure difference according to Example 2. During the circulation in this manner, the valve AV13 is opened and closed in accordance with requirements, so that part of the circulating culture medium can be drained out of the culture system without stopping the supply of culture medium into the culture vessel 11.

Further, by properly switching over the operations of Examples 1 and 2 above described, the culture medium can be replenished from outside the culture system without stopping the supply of culture medium to the culture vessel.

In this manner, continuous feeding of the medium is also possible according to the method of supplying the culture medium in accordance with the invention.

Although the gas-pressure-producing sources have been described as compressor and vacuum pump, other means of obtaining gas pressure, such as a factory pipe line or a compressed gas bomb, could be used.

The culture-medium-supply section and the gas-pressure-supply section could also be variously modified without departing from the invention.

Various culture cells may be used, such as those anchored to the hollow fibre or suspended in the culture chamber. Further, the supply of the culture medium to the culture vessel can be effected in a manner such that the direction of flow of the culture medium in the culture system is fixed or is periodicaly reversed.

For example, when cells that tend to become anchored to the hollow fibre or to be suspended in the chamber are cultivated by supplying culture medium in one direction or two opposite directions, results as shown in Table 1 will be obtained.

## Table 1

| | Direction of culture medium in culture vessel | |
| --- | --- | --- |
| | One fixed direction | Two directions |
| Anchoraged cell | Cell density becomes higher in a zone upstream of the culture medium in the culture vessel. | cell density is uniform over all zone in the culture vessel. |
| Suspension cell | Cell density becomes higher in a zone downstream of the culture medium in the culture vessel. | Cell density is uniform over all zone in the culture vessel. |

With the culture system according to the invention, moreover, various parameters for the operation can easily be controlled within very severe ranges as follows.

(a) Cultivating teperature --- ± 0.1 °C with respect to a set temperature.

(b) DO Value --- ± 0.1 ppm dependent upon proliferation amount of cells

(c) pH Value --- ± 0.1 with respect to a set value.

As can be seen from the above explanation, depending on the nature and method of supply of the culture medium and the system used, the culture medium is fed under gas pressure to the culture vessel. As a result, the culture medium can be supplied into the culture vessel stably and smoothly without any surge or pulse for a long period of time.

Moreover, the system according to the invention is easy to maintain and durable in use because there is no mechanically slidable part and no part with which the culture medium is mechanically in contact. As a result, a maintenance can be carried out without permitting impurities to enter the culture medium, even if the gas pressure supply section fails.

In supplying the culture medium into the culture vessel, the flowing directions of the culture medium in the culture vessel are reversed, so that the cultivating environment can be made uniform throughout the culture vessel so that the cultivating yield rate is improved.

Furthermore, by using different gases for feeding the culture medium or adjusting the components of the gas depending upon the substances being cultivated, the yield ratio can be improved.

When proceeding according to the invention, cultivation can be carried out with an efficiency much higher than that of the prior art to produce cells and substances beneficial for human beings such as immunoglobulin and monoclonal antibodies.

## Claims

1. A method of supplying a culture medium into a culture vessel, characterized in that the culture vessel is connected between two culture medium supply and collection reservoirs that both contain culture medium and a gas phase, the pressure of the gas phase in one such reservoir being different from that in the other whereby culture medium flows from one reservoir to the other, and whereby suitably changing gas pressures in the reservoirs can reverse the direction of flow of the culture medium.

2. A method as claimed in Claim 1, in which the gas pressures are changed by mutual exchange that involves transposing the connexions of gas flow passages connected (a) to the respective gas phases of two pressure chambers each of which has a different gas pressure to the other, and (b) to respective gas phases of the two culture medium supply and collection reservoirs.

3. A method as claimed in Claim 1 or 2 in which at least one component of the gas is changed to control the equilibrium-dissolved amount of gas in the culture medium.

4. A method as claimed in Claim 1 or 2 in which air having oxygen at a concentration higher than that of atmospheric air is used as the gas.

5. A method as claimed in Claim 1 or 2 in which air having oxygen at a concentration lower than that of atmospheric air is used as the gas.

6. A method as claimed in any one of Claims 1 to 5 in which the culture medium is continuously introduced into a culture system including the culture vessel and means for producing the pressure difference, is continuously fed into the culture vessel, and is continuously drained out of the culture system.

7. A method as claimed in any one of Claims 1 to 5 in which the culture medium is introduced into a culture system including the culture vessel and means for producing the pressure difference, and is continuously circulated in the culture system.

8. A method as claimed in Claims 6 and 7 in which the operation for continuously supplying the culture medium into and continuously draining it out of the culture system and the operation for continuously circulating it in the culture system are carried out alternately.

9. A culture system comprising at least one culture vessel (11), a culture medium supply section (21) and a gas pressure supply section (61);

the culture medium supply section (21) having at least two, that is, a first (25a) and a second (25b), culture-medium-supply-and-collection reservoirs, in which each of these reservoirs (25a, 25b) is for containing a gas phase and a culture medium phase and is provided with at least one gas port and at least one culture medium port;

the culture vessel (11) having two culture medium ports, the culture medium port of the first culture-medium-supply-and-collection reservoir (25a) being connected to one of the culture medium ports of the culture vessel (11) through a culture medium flow passage (37 or 39), and the other culture medium port of the culture vessel (11) being connected to the culture medium port of the second culture-medium-supply-and-collection reservoir (25b) through a culture medium flow passage (39 or 37); and

the gas pressure supply section (61) comprising a gas-pressure-producing section (62) for generating gas at at least two different pressures and having at least two gas ports for feeding the two different-pressure gases, one of the gas ports of the gas-pressure-producing section (62) being connected to the gas port of the first reservoir (25a) through a gas flow passage (71 or 72), and the other gas port of the gas-pressure-producing section (62) being connected to the gas port of the second reservoir (25b) through a gas flow passage (72 or 71).

10. A system as claimed in Claim 9, in which the culture-medium-supply section comprises first and second culture-medium-supply-and-collection reservoirs (25a, 25b), a culture-medium-adjusting reservoir (23) provided between the said first and second reservoirs, flow passages for connecting these reservoirs (23, 25a, 25b) and the culture vessel (11), and switching-over means for the flow passages.

11. A system as claimed in Claim 10, in which the culture-medium-adjusting reservoir (23) and the first and second culture-medium-supply-and-collection reservoirs (25a, 25b) are provided with level gauges (LI2, LI1, LI3) for detecting the amount of culture medium in the respective reservoirs.

12. A system as claimed in Claim 10, in which the switching-over means consists of valves (AV1 to 8) in the flow passages and a control section (81) including means for closing and opening the valves.

13. A system as claimed in Claim 10, including a culture-medium-replenishing reservoir (51) connected through a flow passage having a valve (AV12) to said first reservoir (25a) and connected through a flow passage having a valve (AV11) to said second reservoir (25b).

14. A system as claimed in Claim 10, in which the culture-medium-adjusting reservoir (23) is provided with separate means (V3, V4, V9) for supplying oxygen, carbon dioxide and nitrogen to the culture-medium-adjustingreservoir and is also provided with means (50) for supplying an alkaline solution, thereby adjusting the pH and dissolved oxygen content of culture medium in the culture-medium-adjusting reservoir (23).

15. A system as claimed in Claim 14, in which the culture-medium-adjusting reservoir (23) is further provided with a level gauge (23) and a valve (V8) for venting.

16. A system as claimed in Claim 9, in which the gas-pressure-supplying section (61) comprises means (SV2, SV3, 71, 72, 73, 74) for switching over the gas-flow passages to the respective gas ports of the two culture medium supply and collection reservoirs (25a, 25b) of the culture medium supply section (21).

17. A system as claimed in Claim 16, in which the gas-pressure-producing section (62) comprises first and second pressure chambers (63a, 63b) and a compressor (65) for making the pressures in them unequal.

18. A culture system claimed in Claim 17, in which the culture medium supply section is as defined in claim 12 and additionally one of the first and second culture-medium-supply-and-collection reservoirs (25a, 25b) is connected to the first pressure chamber (63a) and the other to the second pressure chamber (63b).

**19.** A system as claimed in Claim 18, in which the connections between the first and second culture-medium-supply-and-collection reservoirs (25a, 25b) and the first and second pressure chambers (63a, 63b) are interchangeable.

**20.** A system as claimed in Claim 16, in which the gas-pressure-producing section (62) comprises pressure-supply means and pressure-regulating means for regulating pressure from it, and a lower side of the gas-pressure-producing section (62) is directly opened to atmospheric pressure.

**21.** A system as claimed in Claim 20, in which the pressure supply means is a high-pressure gas bomb.

**22.** A system as claimed in claim 9, in which the gas-pressure-supply section (61) comprises a gas-pressure-producing section, a negative-gauge-pressure-producing section (171), gas flow passages provided between the gas-pressure-producing section and the culture-medium-supply section and between the gas-pressure-producing section and the negative-gauge-pressure-producing section (171), and switching-over means for switching over the gas flow passages.

**23.** A system as claimed in claim 22, in which the negative-gauge-pressure producing section (171) comprises a vacuum pump (173), a third pressure chamber (63c) for storing negative-gauge-pressure gas, gas flow passages between the vacuum pump (173), the third pressure chamber (63c) and other components in the gas-pressure-supply section, and switching-over means for switching over the flow passages.

**24.** A system as claimed in Claim 9, in which the culture medium supply section comprises first and second culture-medium-supply-and-collection reservoirs (25a, 25b), a culture-medium-adjusting reservoir (23) provided between these reservoirs (25a, 25b), a bypass between the culture-medium-adjusting reservoir (23) and the first and second culture-medium-supply-and-collection reservoirs (25a, 25b), flow passages for connecting these tanks and the bypass, and switching-over means for the flow passages.

**25.** A system as claimed in Claim 24, in which the bypass comprises first and second flow passages (36, 41) having valves (AV7, AV3) respectively, and connected in series with each other, third and fourth flow passages (38, 44) having valves (AV8, AV6) respectively, connected in series with each other, the two pairs of passages (36, 41) and (38, 44) being themselves connected in parallel in a flow passage (112, 113) of the bypass means, a fifth flow passage having a valve (AV9) and connecting one end of the culture vessel (11) and the join between the first and second flow passages (36, 41), and a sixth flow passage having a valve (AV10) and connecting the other end of the culture vessel (11) and the join between the third and fourth flow passages (38, 44).

**Patentansprüche**

**1.** Verfahren zur Zuführung eines Kulturmediums in einen Kulturbehälter, **dadurch gekennzeichnet,** daß der Kulturbehälter zwischen zwei Kulturmediumzuführungs- und -sammelbehältern, die beide Kulturmedium und eine Gasphase enthalten, zwischengeschaltet wird, wobei der Druck der Gasphase in einem solchen Behälter verschieden von jenem in dem anderen ist, wodurch Kulturmedium von einem Behälter zum anderen fließt, und wobei eine geeignete Veränderung der Gasdrücke in den Behältern die Fließrichtung des Kulturmediums umkehren kann.

**2.** Verfahren nach Anspruch 1, bei dem die Gasdrücke durch wechselseitigen Austausch verändert werden, der ein Umstellen der Verbindungen der Gasflußdurchgänge einschließt, welche (a) mit den betreffenden Gasphasen von zwei Druckkammern, von denen jede einen anderen Gasdruck als die andere aufweist, und (b) mit den betreffenden Gasphasen der beiden Kulturmediumzuführungs- und -sammelbehälter verbunden sind.

**3.** Verfahren nach Anspruch 1 oder 2, bei dem wenigstens eine Komponente des Gases verändert wird, um die im Gleichgewicht gelöste Gasmenge in dem Kulturmedium zu steuern.

**4.** Verfahren nach Anspruch 1 oder 2, bei dem als da Gas Luft mit Sauerstoff in einer höheren Konzentration als jene von atmosphärischer Luft verwendet wird.

5. Verfahren nach Anspruch 1 oder 2, bei dem als das Gas Luft mit Sauerstoff in einer niedrigeren Konzentration als jene von atmosphärischer Luft verwendet wird.

6. Verfahren nach einem der Ansprüche 1 bis 5, bei dem da Kulturmedium kontinuierlich in ein Kultursystem einschließlich des Kulturbehälters und Einrichtungen zur Erzeugung des Druckunterschiedes eingeführt wird, kontinuierlich in den Kulturbehälter eingespeist wird und kontinuierlich aus dem Kultursystem abgelassen wird.

7. Verfahren nach einem der Ansprüche 1 bis 5, bei weichem das Kulturmedium in ein Kultursystem einschließlich des Kulturbehälters und Einrichtungen zur Erzeugung des Druckunterschiedes eingeführt und kontinuierlich in dem Kultursystem zirkuliert wird.

8. Verfahren nach Anspruch 6 und 7, bei welchem der Betrieb für kontinuierliche Zuführung des Kulturmediums in das Kultursystem und kontinuierliches Ablassen desselben aus dem Kultursystem und der Betrieb für kontinuierliches Zirkulieren desselben in dem Kultursystem alternierend durchgeführt werden.

9. Kultursystem mit wenigstens einem Kulturbehälter (11), einem Kulturmediumzuführungs- abschnitt (21) und einem Gasdruckzuführungsabschnitt (61),
wobei der Kulturmediumzuführungsabschnitt (21) wenigstens zwei, d. h. einen ersten (25a) und einen zweiten (25b), Kulturmediumzuführungs- und -sammelbehälter hat, worin jeder dieser Behälter (25a, 25b) eine Gasphase und eine Kulturmediumphase enthalten kann und mit wenigstens einer Gasöffnung und wenigstens einer Kulturmediumöffnung versehen ist,
der Kulturbehälter (11) zwei Kulturmediumöffnungen hat, wobei die Kulturmediumöffnung des ersten Kulturmediumzuführungs- und -sammelbehälters (25a) mit einer der Kulturmediumöffnungen des Kulturbehälters (11) über einen Kulturmediumflußdurchgang (37 oder 39) verbunden ist und die andere Kulturmediumöffnung des Kulturbehälters (11) mit der Kulturmediumöffnung des zweiten Kulturmediumzuführungs- und -sammelbehälters (25b) über einen Kulturmediumflußdurchgang (39 oder 37) verbunden ist, und
der Gasdruckzuführungsabschnitt (61) einen Gasdruck erzeugenden Abschnitt (62) zur Erzeugung von Gas mit wenigstens zwei unterschiedlichen Drücken umfaßt und wenigstens zwei Gasöffnungen zur Einspeisung der beiden Gase mit unterschiedlichem Druck hat, wobei eine der Gasöffnungen des Gasdruck erzeugenden Abschnittes (62) mit der Gasöffnung des ersten Behälters (25a) über einen Gasflußdurchgang (71 oder 72) verbunden ist und die andere Gasöffnung des Gasdruck erzeugenden Abschnittes (62) mit der Gasöffnung des zweiten Behälters (25b) über einen Gasflußdurchgang (72 oder 71) verbunden ist.

10. System nach Anspruch 9, in weichem der Kulturmediumzuführungsabschnitt erste und zweite Kulturmediumzuführungs- und -semmelbehälter (25a, 25b), einen zwischen dem ersten und zweiten Behälter vorgesehenen Kulturmediumeinstellungsbehälter (23), Fließdurchgänge zur Verbindung dieser Behälter (23, 25a, 25b) mit dem Kulturbehälter (11) und Umschalteinrichtungen für die Fließdurchgänge umfaßt.

11. System nach Anspruch 10, in welchem der Kulturmediumelnstellbehälter (23) und der erste und zweite Kulturmediumzuführungs- und -sammelbehälter (25a, 25b) mit Pegelmeßeinrichtungen (LI2, LI1, LI3) zur Bestimmung der Kulturmediummenge in den betreffenden Behälterm versehen sind.

12. System nach Anspruch 10, in weichem die Umschalteinrichtungen aus Ventilen (AV1 bis 8) in den Fließdurchgängen und einem Steuerabschnitt (81) einschließlich Einrichtungen zum Schließen und Öffnen der Ventile bestehen.

13. System nach Anspruch 10 einschließlich eines Kulturmediumnachfüllbehälters (51), der über einen Fließdurchgang mit einem Ventil (AV12) mit dem ersten Behälter (25a) verbunden ist und über einen Fließdurchgang mit einem Ventil (AV11) mit dem zweiten Behälter (25b) verbunden ist.

14. System nach Anspruch 10, in weichem der Kulturmediumeinstellbehälter (23) mit getrennten Einrichtungen (V3, V4, V9) für die Zuführung von Sauerstoff, Kohlendioxid und Stickstoff zu dem Kulturmediumeinstellbehälter versehen ist und auch mit Einrichtungen (50) zur Zuführung einer alkalischen Lösung

15

versehen ist, um so den pH-Wert und den Gehalt an gelöstem Sauerstoff des Kulturmediums in dem Kulturmediumeinstellbehälter(23) einzustellen.

**15.** System nach Anspruch 14, in welchem der Kulturmediumeinstellbehälter (23) weiterhin mit einer Pegelmeßeinrichtung (23) und einem Ventil (V8) zur Lüftung versehen ist.

**16.** System nach Anspruch 9, in welchem der Gasdruckzuführungsabschnitt (61) Einrichtungen (SV2, SV3, 71, 72, 73, 74) zum Umschalten der Gasflußdurchgänge zu den betreffenden Gasöffnungen der beiden Kulturmediumzuführungs- und -sammelbehälter (25a, 25b) des Kulturmediumzuführungsabschnittes (21) aufweist.

**17.** System nach Anspruch 16, in welchem der Gasdruck erzeugende Abschnitt (81) erste und zweite Druckkammern (63a, 63b) und einen Kompressor (65), um die Drücke in ihnen ungleich zu machen, umfaßt.

**18.** Kultursystem nach Anspruch 17, in welchem der Kulturmediumzuführungsabschnitt wie in Anspruch 12 definiert ist und außerdem einer der ersten und zweiten Kulturmediumzuführungs- und -sammelbehälter (25a, 25b) mit der ersten Druckkammer (63a) und der anderen mit der zweiten Druckkammer (63b) verbunden ist.

**19.** System nach Anspruch 18, in weichem die Verbindungen zwischen dem ersten und zweiten Kulturmediumzuführungs- und -sammelbehälter (25a, 25b) und der ersten und zweiten Druckkammer (63a, 63b) untereinander austauschbar sind.

**20.** System nach Anspruch 16, in welchem der Gasdruck erzeugende Abschnitt (61) Druckzuführungseinrichtungen und Druckreguliereinrichtungen zur Regulierung des Druckes von ihm umfaßt und eine Unterseite des Gasdruck erzeugenden Abschnittes (61) direkt zu atmosphärischem Druck geöffnet ist.

**21.** System nach Anspruch 20, in welchem die Druckzuführungseinrichtung eine Hochdruckgasbombe ist.

**22.** System nach Anspruch 9, in welchem der Gasdruckzuführungsabschnitt (61) einen Gasdruck erzeugenden Abschnitt, einen Negativmanometerdruck erzeugenden Abschnitt (171), Gasflußdurchgänge zwischen dem Gasdruck erzeugenden Abschnitt und dem Kulturmediumzuführungsabschnitt sowie zwischen dem Gasdruck erzeugenden Abschnitt und dem Negativmanometerdruck erzeugenden Abschnitt (171) sowie Umschalteinrichtungen zum Umschalten der Gasflußdurchgänge aufweist.

**23.** System nach Anspruch 22, in weichem der Negativmanometerdruck erzeugende Abschnitt (171) eine Vakuumpumpe (173), eine dritte Druckkammer (63c) zur Bevorratung von Negativmanometerdruckgas, Gasflußdurchgänge zwischen der Vakuumpumpe (173), der dritten Druckkammer (63c) und anderen Teilen des Gasdruckzuführungsabschnittes sowie Umschalteinrichtungen zum Umschalten der Fließdurchgänge umfaßt.

**24.** System nach Anspruch 9, in welchem der Kulturmediumzuführungsabschnitt erste und zweite Kulturmediumzuführungs- und -sammelbehälter (25a, 25b), einen Kulturmediumeinstellbehälter (23) zwischen diesen Behältern (25a, 25b), einen Bypass zwischen dem Kulturmediumeinstellbehälter (23) und den ersten und zweiten Kulturmediumzuführungs-und -sammelbehältern (25a, 25b), Fließdurchgänge zur Verbindung dieser Behälter mit dem Bypass und Umschalteinrichtungen für die Fließdurchgänge umfaßt.

**25.** System nach Anspruch 24, in welchem der Bypass erste und zweite Fließdurchgänge (36, 41) jeweils mit Ventilen (AV7, AV3) und in Reihe miteinander verbunden, dritte und vierte Fließdurchgänge (38, 44) jeweils mit Ventilen (AV8, AV6), in Reihe miteinander verbunden, wobei die beiden Paare von Durchgängen (36, 41) und (38, 44) selbst parallel in einem Fließdurchgang (112, 113) der Bypasseinrichtung verbunden sind, einen fünften Fließdurchgang mit einem Ventil (AV9), der ein Ende des Kulturbehälters (11) und die Verbindung zwischen den ersten und zweiten Fließdurchgängen (36, 41) miteinander verbindet, und einen sechsten Fließdurchgang mit einem Ventil (AV10), der das andere Ende des Kulturbehälters (11) und die Verbindung zwischen den dritten und vierten Fließdurchgängen (38, 44) miteinander verbindet, aufweist.

EP 0 263 634 B1

**Revendications**

1. Procédé de délivrance d'un milieu de culture dans un récipient de culture caractérisé en ce que le récipient de culture est raccordé entre deux réservoirs de délivrance et de collecte de milieu de culture qui contiennent à la fois un milieu de culture et une phase gazeuse, la pression de la phase gazeuse dans l'un des réservoirs étant différente de celle dans l'autre, ce par quoi le milieu de culture s'écoule d'un réservoir vers l'autre, et ce par quoi des changements appropriés de pression gazeuse dans les réservoirs peuvent inverser le sens d'écoulement du milieu de culture.

2. Procédé selon la revendication 1, dans lequel les pressions gazeuses sont modifiées par permutation ce qui entraîne la transposition des raccordements des conduits d'écoulement de gaz reliés (a) aux phases gazeuses respectives de deux chambres de pression, dont chacune a une pression gazeuse différente de l'autre, et (b) a des phases gazeuses respectives de deux réservoirs de délivrance et de collecte de milieu de culture.

3. Procédé selon la revendication 1 ou 2, dans lequel au moins l'un des composants du gaz est modifié pour commander la quantité dissoute d'équilibre de gaz dans le milieu de culture.

4. Procédé selon la revendication 1 ou 2, dans lequel de l'air contenant de l'oxygène en une concentration plus élevée que celle de l'air atmosphérique est utilisé comme gaz.

5. Procédé selon la revendication 1 ou 2, dans lequel de l'air contenant de l'oxygène en une concentration plus faible que celle de l'air atmosphérique est utilisé comme gaz.

6. Procédé selon l'une quelconque des revendications 1 à 5, dans lequel le milieu de culture est introduit de façon continu dans un système de culture incluant le récipient de culture et des moyens pour produire la différence de pression, est délivré en continu dans le récipient de culture, et est soutiré en continu hors du système de culture.

7. Procédé selon l'une quelconque des revendications 1 à 5, dans lequel le milieu de culture est introduit dans un système de culture comportant le récipient de culture et des moyens pour produire la différence de pression et est mis en circulation en continu dans le système de culture.

8. Procédé selon les revendications 6 et 7, dans lequel la l'opération pour délivrer en continu le milieu de culture dans le système de culture et pour le soutirer de celui-ci et l'opération pour le faire circuler en continu dans le système de culture sont exécutées de façon alternée.

9. Système de culture comprenant au moins un récipient de culture (11), une section de délivrance de milieu de culture (21) et une section de fourniture de pression gazeuse (61) ;
   la section de délivrance de milieu de culture (21) ayant au moins deux, c'est-à-dire un premier (25a) et un second (25b), réservoirs de délivrance et de collecte de milieu de culture, où chacun de ces réservoirs (25a, 25b) sert à contenir une phase gazeuse et une phase milieu de culture et est pourvu d'au moins un orifice de gaz et d'au moins un orifice de milieu de culture ;
   le récipient de culture (11) comportant deux orifices de milieu de culture, l'orifice de milieu de culture du premier réservoir de délivrance et de collecte de milieu de culture (25a) étant relié à l'un des orifices de milieu de culture du récipient de culture (11) par un conduit d'écoulement de milieu de culture (37 ou 39), et l'autre orifice de milieu de culture du récipient de culture (11) étant relié à l'orifice de milieu de culture du second réservoir de délivrance et de collecte de milieu de culture (25b) par un conduit d'écoulement de milieu de culture (39 ou 37) ; et
   la section de fourniture de pression gazeuse (61) comprenant une section de production de pression gazeuse (62) pour produire du gaz à au moins deux pressions différentes et comportant au moins deux orifices de gaz pour délivrer les deux pressions gazeuses différentes, l'un des orifices de gaz de la section de production de pression gazeuse (62) étant relié à l'orifice de gaz du premier réservoir (25a) par un conduit d'écoulement de gaz (71 ou 72), et l'autre orifice de gaz de la section de production de pression gazeuse (62) étant relié à l'orifice de gaz du second réservoir (25b) par un conduit d'écoulement de gaz (72 ou 71).

17

EP 0 263 634 B1

**10.** Système selon la revendication 9, dans lequel la section de délivrance de milieu de culture comprend des premier et second réservoirs de délivrance et de collecte de milieu de culture (25a, 25b), un réservoir d'ajustement de milieu de culture (23) placé entre lesdits premier et second réservoirs, des conduits d'écoulement pour relier ces réservoirs (23, 25a, 25b) et le récipient de culture (11) et des moyens de permutation pour les conduits d'écoulement.

**11.** Système selon la revendication 10, dans lequel le réservoir d'ajustement de milieu de culture (23) et les premier et second réservoirs de délivrance et de collecte de milieu de culture (25a, 25b) sont munis de jauges de niveau (LI2, LI1, LI3) pour détecter la quantité de milieu de culture dans les réservoirs respectifs.

**12.** Système selon la revendication 10, dans lequel le moyen de permutation est constitué de vannes (AV1 à 8) dans les conduits d'écoulement et d'une section de commande (81) comportant des moyens pour fermer et ouvrir les vannes.

**13.** Système selon la revendication 10, comportant un réservoir de recomplètement de niveau de culture (51) raccordé par un conduit d'écoulement comportant une vanne (AV12) audit premier réservoir (25a) et raccordé par un conduit d'écoulement comportant une vanne (AV11) audit second réservoir (25b).

**14.** Système selon la revendication 10, dans lequel le réservoir d'ajustement de milieu de culture (23) est pourvu de moyens distincts (V3, V4, V9) pour délivrer de l'oxygène, du dioxyde de carbone et de l'azote au réservoir d'ajustement de milieu de culture et est également pourvu de moyens (50) pour délivrer une solution alcaline, pour ajuster par ce moyen le pH et la teneur en oxygène dissout du milieu de culture dans le réservoir d'ajustement de milieu de culture (23).

**15.** Système selon la revendication 14, dans lequel le réservoir d'ajustement de milieu de culture (23) est en outre pourvu d'une jauge de niveau (23) et d'une vanne (V8) pour mise à l'air libre.

**16.** Système selon la revendication 9, dans lequel la section de fourniture de pression gazeuse (61) comprend des moyens (SV2, SV3, 71, 72, 73, 74) pour permuter les conduits d'écoulement de gaz vers les orifices de gaz respectifs des deux réservoirs d'alimentation et de collecte de milieu de culture (25a, 25b) de la section de délivrance de milieu de culture (21).

**17.** Système selon la revendication 16, dans lequel la section de production de pression gazeuse (62) comprend des première et seconde chambres de pression (63a, 63b) et un compresseur (65) pour rendre leurs pressions inégales.

**18.** Système selon la revendication 17, dans lequel la section de délivrance de milieu de culture, telle qu'elle est définie dans la revendication 12, et en plus l'un des premier et second réservoirs de délivrance et de collecte de milieu de culture (25a, 25b), sont raccordés à la première chambre de pression (63a) et l'autre à la seconde chambre de pression (63b).

**19.** Système selon la revendication 18, dans lequel les liaisons entre les premier et second réservoirs de délivrance et de collecte de milieu de culture (25a, 25b) et les première et seconde chambres de pression (63a, 63b) peuvent être permutées.

**20.** Système selon la revendication 16, dans lequel la section de production de pression gazeuse (62) comprend des moyens de fourniture de pression et des moyens de régulation de pression pour réguler la pression qui en est issue, et dans lequel un côté inférieur de la section de production de pression gazeuse (62) est directement ouvert à la pression atmosphérique.

**21.** Système selon la revendication 20, dans lequel les moyens d'alimentation en pression sont constitués par une bombonne de gaz à haute pression.

**22.** Système selon la revendication 9, dans lequel la section de fourniture de pression gazeuse (61) comprend une section de production de section gazeuse, une section de production de pression gazeuse négative (171), des conduits d'écoulement de gaz disposés entre la section de production de pression gazeuse et la section de délivrance de milieu de culture et entre la section de production de

18

pression gazeuse et la section de production de pression gazeuse négative (171), et des moyens de permutation pour permuter les conduits d'écoulement.

23. Système selon la revendication 22, dans lequel la section de production de pression gazeuse négative (171) comprend une pompe à vide (173), une troisième chambre de pression (63c) pour emmagasiner du gaz sous pression négative, des conduits d'écoulement de gaz entre la pompe à vide (173), la troisième chambre de pression (63c) et d'autres composants dans la section de fourniture de pression gazeuse, et des moyens de permutation pour permuter les conduits d'écoulement.

24. Système selon la revendication 9, dans lequel la section de délivrance de milieu de culture comprend des premier et second réservoirs de délivrance et de collecte de milieu de culture (25a, 25b), un réservoir d'ajustement de milieu de culture (23) placé entre ces réservoirs (25a, 25b), une dérivation entre le réservoir d'ajustement de milieu de culture (23) et les premier et second réservoirs de délivrance et de collecte de milieu de culture (25a, 25b), des conduits d'écoulement pour relier ces réservoirs et la dérivation, et des moyens de permutation pour les conduits d'écoulement.

25. Système selon la revendication 24, dans lequel la dérivation comprend des premier et deuxième conduits d'écoulement (36, 41) comportant, respectivement, des vannes (AV7, AV3), et raccordées en série l'une avec l'autre, des troisième et quatrième conduits d'écoulement (38, 44) comportant, respectivement, des vannes (AV8, AV6) raccordées en série l'une avec l'autre, les deux paires de conduits (36, 41) et (38, 44) étant elles mêmes raccordées en parallèle dans un conduit d'écoulement (112, 113) des moyens de dérivation, un cinquième conduit d'écoulement comportant une vanne (AV9) et reliant l'une des extrémités du récipient de culture (11) et la jonction entre les premier et deuxième conduits d'écoulement (36, 41), et un sixième conduit d'écoulement comportant une vanne (AV10) et reliant l'autre extrémité du récipient de culture (11) et la jonction entre les troisième et quatrième conduits d'écoulement (38, 44).

# FIG. 1

FIG.2A

# FIG_2B

EP 0 263 634 B1

*FIG. 3*

# FIG_4A

**FIG.4B**